# EUROPÄISCHE PATENTSCHRIFT

(11) **EP 3 624 870 B1**
(45) Veröffentlichungstag und Bekanntmachung des Hinweises auf die Patenterteilung: **11.09.2024**
(21) Anmeldenummer: 18728534.1
(22) Anmeldetag: 16.05.2018
(51) Int. Cl.: A61M 1/28

(54) **PERITONEALDIALYSESYSTEM**
PERITONEAL DIALYSIS SYSTEM
SYSTÈME DE DIALYSE PÉRITONÉALE

(30) Priorität: 16.05.2017 DE 102017110573
(43) Veröffentlichungstag der Anmeldung: 25.03.2020
(73) Patentinhaber: Fresenius Medical Care Deutschland GmbH, 61352 Bad Homburg (DE)
(72) Erfinder: WOLF, Klaus, 97450 Muedesheim (DE); HASSLER, Manuel, 61440 Oberursel (DE)
(74) Vertreter: Herrmann, Uwe
(86) Internationale Anmeldenummer: PCT/EP2018/062716
(87) Internationale Veröffentlichungsnummer: WO 2018/210926

(56) Entgegenhaltungen:
- EP-A1- 0 950 422
- CN-U- 205 126 976
- DE-C1- 10 023 600
- DE-U1- 202013 000 017
- US-A- 5 782 796
- US-A1- 2002 077 608
- US-A1- 2008 277 219
- US-B1- 6 284 139

## Beschreibung

Die vorliegende Erfindung betrifft ein Peritonealdialysesystem umfassend wenigstens ein Peritonealdialysegerät sowie wenigstens ein Organisationssystem, das ausgebildet ist, das Peritonealdialysegerät und/oder ein oder mehrere funktionale Elemente, die mit dem Peritonealdialysegerät in Verbindung stehen oder mit diesem verbindbar sind, zu tragen.

Die Peritonealdialyse wird von Patienten an einem typischen Behandlungsort durchgeführt, bei dem es sich nicht zwingend um eine Praxis oder Klinik handeln muss. Häufig erfolgt die Peritonealdialyse zu Hause bzw. im heimischen Schlafzimmer. Die Patienten nutzen üblicherweise einen sogenannten Porter, d.h. ein Organisationssystem, das dazu dient, um das Peritonealdialysegerät, die benötigten Lösungsbeutel, das Drainagesystem sowie die Patientenkonnektion zu organisieren und so gut wie möglich zu unterstützen.

Die vorliegende Erfindung ist nicht auf eine bestimmte Art der PD-Behandlung bzw. nicht auf ein bestimmtes PD-Gerät beschränkt. Es kann sich beispielsweise um ein APD-Gerät handeln, bei dem alle oder mehrere Schritte, wie z.B. das Öffnen und Schließen von Klemmen, das Starten und Stoppen von Pumpen automatisiert erfolgt.

Es kann sich um ein gravimetrisches Gerät handeln, bei der Fluss der Lösungen durch Schwerkraft bewirkt wird und/oder um ein Gerät mit einer oder mehreren Pumpen, die die Aufgabe haben, die Lösung zu fördern.

Die DE 100 23 600 C1 offenbart ein Gepäckstück, das alle zur Durchführung einer Peritonealdialyse notwendigen Hilfsmittel enthält, wobei es an einer Seitenfläche vertikal ausziehbare Teleskopstangen aufweist, die sowohl einzeln als Infusionsständer verwendet als auch miteinander zu einem Haltegriff verbunden werden können.

Aus der US 6 284 139 B1 ist ein Verfahren zur Erleichterung der Peritonealdialyse bei einem Patienten bekannt, wobei das Verfahren die folgenden Schritte umfasst: Bereitstellung eines Trägers, der mindestens eine Ablage, mindestens einen Ständer zum Halten der mindestens einen Ablage in einer erhöhten Position über einer darunter liegenden Fläche, auf der die Halterung angeordnet ist, und mindestens ein Rad umfasst; Anordnen einer selbständigen in sich geschlossenen Peritonealdialyseeinheit auf dem mindestens einen Regal; Durchführen einer Peritonealdialyse an dem Patienten unter Verwendung der in sich geschlossenen Peritonealdialyseeinheit.

Die CN 205 126 976 U offenbart eine zur Peritonealdialyse geeignete Vorrichtung, die einen Tropfständer umfasst, wobei die Oberseite des Tropfständers mit einem Haken versehen ist, der jeweils zum Aufhängen eines Beutels für Peritonealdialyseflüssigkeit und einer Wägeeinrichtung verwendet wird, und die Wägeeinrichtung zum Wiegen des Gewichts des Beutels für Peritonealdialyseflüssigkeit und der Abfallflüssigkeit der Dialyse dient.

Sind die Patienten unterwegs, wie beispielweise im Urlaub, steht dieser Porter häufig nicht zur Verfügung oder nimmt sehr viel Platz beim Reisen ein, so dass eine Reise gänzlich unmöglich wird oder jedenfalls nur mit größeren Umständen erfolgen kann.

Der vorliegenden Erfindung liegt somit die Aufgabe zugrunde, ein Peritonealdialysesystem der eingangs genannten Art dahingehend weiterzubilden, dass für den Peritonealdialyse-Patienten auch auf Reisen eine gute Vorbereitung der Behandlung bzw. eine gute Organisation des Peritonealdialysegeräts, der Verbrauchsmaterialien sowie der Patientenkonnektion und -diskonnektion möglich ist.

Diese Aufgabe wird durch ein Peritonealdialysesystem mit den Merkmalen des Anspruchs 1 gelöst.

Danach ist vorgesehen, dass das Organisationssystem zu Transport- oder Lagerzwecken zusammenlegbar ist und zum Zwecke der Durchführung der Peritonealdialysebehandlung ausgehend von dem zusammengelegten Zustand in seinen Funktionszustand aufbaubar ist, wobei das Organisationssystem ein oder mehrere Tragelemente zum Tragen des oder der funktionalen Elemente, wie z.B. von Lösungsbeuteln etc. aufweist.

Der vorliegenden Erfindung liegt der Gedanke zugrunde, das Organisationssystem so auszubilden, dass es vom Patienten zusammengelegt werden kann und damit leicht auf Reisen mitgenommen werden kann. Vor Ort kann der Patienten das zusammengelegte Organisationssystem wieder auseinander falten, so dass es für die anstehende Peritonealdialysebehandlung zur Verfügung steht.

Vorzugsweise handelt es sich bei dem Peritonealdialysegerät um ein APD-Gerät, d.h. um ein Gerät, das eine oder mehrere Pumpen zur Förderung des frischen und/oder gebrauchten Dialysats aufweist. Die Erfindung ist jedoch nicht auf derartige Peritonealdialysegeräte beschränkt.

Das Organisationssystem ist vorzugsweise leicht, so dass es gut zu transportieren ist. Es ist zusammenfaltbar, so dass sich eine äußeren Abmessungen bzw. sein Volumen gegenüber dem aufgebauten Funktionszustand, in dem es für die Peritonealdialyse zur Verfügung steht, verringern.

Bei dem oder den funktionalen Elementen, die durch das Organisationssystem getragen, fixiert, gehalten etc. werden, kann es sich um ein beliebiges Element handeln, das der Patient im Rahmen der Peritonealdialysebehandlung benötigt.

Als nicht abschließende Aufzählung kommen beispielsweise Lösungsbeutel, die die frische Peritonealdialyselösung enthalten, Drainagebeutel zur Aufnahme des benutzten Dialysats, Schläuche oder Schlauchsysteme zur Führung des Dialysats, Ventile zur Steuerung des Dialysatflusses, Organizer zur Aufnahme von Konnektoren des Schlauchsets mit weiteren Leitungen und dem Patientenanschluss, Konnektoren etc. in Betracht.

Das Organisationssystem kann beispielsweise zur Halterung der Lösungsbeutel einen oder mehrere integrierte Haken oder sonstige Halterungsmittel aufweisen. Alternativ oder zusätzlich kann das Organisationssystem eine oder mehrere integrierte Aufnahmen oder sonstige Halterungsmittel für den Organzier und/oder für den Patientenkonnektor aufweisen.

Denkbar ist es, dass das Organisationssystem selbsttragend ist oder durch den Transportkoffer, in dem das Peritonealdialysegerät transportiert wird und/oder durch das Peritonealdialysegerät getragen bzw. stabilisiert wird.

Erfindungsgemäß besteht das Organisationssystem aus einer leichten, faltbaren Konstruktion, die im zusammengefalteten Zustand wenig Platz einnimmt und in einem Transportkoffer, in dem das Peritonealdialysegerät transportiert wird, Platz findet.

Das Organisationssystem kann als reines Gestell ausgebildet sein oder eine oder mehrere Wandungen aufweisen, die als Verkleidung des Peritonealdialysegerätes und/oder von dessen Transportkoffer dienen.

Das Organisationssystem weist wenigstens eine Abstellfläche auf, auf der das Peritonealdialysegerät abstellbar ist. Diese Stellfläche ist höhenverstellbar, so dass das Gerät einfach auf der gewünschten Höhe relativ zum Patienten positioniert werden kann.

Erfingungsgemäß weist das Peritonealdialysesystem einen Transportkoffer auf, in dem sich das Peritonealdialysegerät beim Transport befindet. Dabei kann eine Fläche des Transportkoffers als Abstellfläche für das Peritonealdialysegerät bei dessen Gebrauch dienen.

In einer bevorzugten Ausgestaltung der Erfindung weist das Organisationssystem eine oder mehrere Aufnahmen oder Aufhängungen für den oder die die frische Dialyselösung enthaltende Lösungsbeutel auf, die im Rahmen der Peritonealdialysebehandlung benötigt werden.

Das Organisationssystem kann eine oder mehrere Aufnahmen oder Aufhängungen für den oder die Drainagebeutel aufweisen, in die die gebrauchte Dialyselösung abgelassen wird. Vorzugsweise ist jedoch vorgesehen, dass der oder die Drainagebeutel in dem Transportkoffer aufgenommen sind, in dem das Peritonealdialysegerät transportiert wird.

Denkbar ist es weiterhin, dass das Organisationssystem Rollen aufweist, mittels derer das Organisationssystem verfahrbar ist, so dass es in die für die Behandlung günstigste Position verfahren werden kann.

Alternativ oder zusätzlich kann auch der Transportkoffer, in dem sich das Peritonealdialysegerät während des Transports befindet Rollen aufweisen, die vorzugsweise in den Koffer integriert sind. Dient der Transportkoffer als Tisch für das Peritonealdialysegerät, ist dies besonders sinnvoll, da das auf dem Transportkoffer befindliche Peritonealdialysegerät so ohne weiteres in die gewünschte Position verfahren werden kann.

Um eine optimale Höhe insbesondere für Lösungs- und Drainagebeutel breitstellen zu können, kann weiterhin vorgesehen sein, dass das Organisationssystem insgesamt oder wenigstens teilweise höhenverstellbar ausgeführt ist.

Das Organisationssystem ist vorzugsweise derart ausgebildet, dass es in seinem Funktionszustand, d.h. im nicht zusammengelegten Zustand wenigstens eine Abstellfläche oberhalb des Peritonealdialysegerätes aufweist. Auf dieser kann beispielsweise Zubehör abgelegt werden. Auch kann das Organisationssystem eine Fläche aufweisen, die vorzugsweise ebenfalls oberhalb des Peritonealdialysegerätes angeordnet ist und die z.B. zum Öffnen eines Doppelkammerbeutels dient.

Das Organisationssystem kann an oder in dem Transportkoffer integriert sein oder das Organisationssystem kann als von dem Transportkoffer getrennte Einheit ausgeführt sein.

Der Transportkoffer kann so ausgebildet sein, dass an dem Transportkoffer ein oder mehrere Stangen mit Halterungen und/oder sonstige Aufnahmen bzw. Halterungen für ein oder mehrere funktionale Elemente, insbesondere für Lösungsbeutel angeordnet sind. Somit kann alternativ oder zusätzlich zu dem Organisationssystem auch der Transportkoffer selbst dazu herangezogen werden, Lösungsbeutel, Organizer etc. zu halten bzw. zu tragen.

In einer weiteren bevorzugten Ausgestaltung der Erfindung weist der Transportkoffer wenigstens eine Durchführung für zumindest einen Schlauch und wenigstens eine Aufnahme für wenigstens ein funktionales Element, insbesondere für einen Drainagebeutel auf. Die Drainage erfolgt somit in den Koffer bzw. in den oder die darin befindlichen Drainagebeutel.

An dieser Stelle wird darauf hingewiesen, dass der Begriff "Beutel" stellvertretend für jedes beliebige Behältnis steht, in dem eine Dialyselösung aufgenommen werden kann. Es kann, muss sich daher nicht um ein Behältnis mit flexiblen Wandungen handeln, von der Erfindung und von dem Begriff "Beutel" ist beispielsweise auch die Verwendung einer Box mit starren Wandungen umfasst.

Grundsätzlich kann vorzugsweise vorgesehen sein, dass in dem Koffer eine schräge Einlage angeordnet ist sowie eine Schlauchdurchführung zur Durchführung eines Schlauches von dem auf der Schräge befindlichen Drainagebeutel zu dem Peritonealdialysegerät. Alternativ oder zusätzlich können auf der Schräge auch Lösungsbeutel mit frischer Dialyselösung angeordnet sein. Diese kann dann mittels des durch die Schlauchdurchführung geführten Schlauches zum Patienten geleitet werden. Die Schräge ist vorzugsweise derart ausgeführt, dass der Beutel mit der frischen Dialyselösung vollständig ausläuft, d.h. dass die frische Dialyselösung vollständig zum Patienten gelangt.

Insbesondere bei Geräten mit Pumpsystemen kann auch der Dialyselösungsbeutel im Koffer untergebracht werden. Die schräge Unterlage gewährleistet ein Vollständiges Auslaufen des Lösungsbeutels.

Das Organisationssystem umgibt als Verkleidung in seinem Funktionszustand, d.h. im nicht zusammengelegten Zustand das Peritonealdialysegerät und ggf. den Transportkoffer teilweise oder vollständig.

Das gesamte Peritonealdialysesystem oder zumindest ein Teil von diesem kann somit verkleidet werden bzw. optisch in den Behandlungsraum integriert werden.

Denkbar ist es, dass das Organisationssystem in unterschiedlichen Materialen und/oder mit unterschiedlichen Oberflächen verfügbar ist, so dass dieses an die örtlichen Gegebenheiten und Wünsche des Patienten angepasst werden kann.

Vorzugsweise verkleidet das Organisationssystem das Peritonealdialysegerät und den Transportkoffer, so dass das Peritonealdialysegerät während und außerhalb der Behandlung nicht als medizinisches Gerät zu Hause bzw. in dem Behandlungsraum wahrgenommen wird.

Das Organisationssystem kann Aufnahmebereiche aufweisen, in den z.B. Disposable bzw. Einmalartikel, wie z.B. das Schlauchsystem, Desinfektionskappen, Desinfektionsmittel etc. aufgenommen werden können.

Die vorliegende Erfindung betrifft des Weiteren ein Verfahren zur Vorbereitung eines Verfahrens zur Vorbereitung einer Peritonealdialysebehandlung, wobei zur Vorbereitung ein Peritonealdialysesystem gemäß einem der Ansprüche 1 bis 7 verwendet wird. Das Verfahren betrifft somit nicht die Behandlung als solche, sondern deren Vorbereitung.

Dies kann z.B. den Aufbau des Organisationssystems bzw. die Bereitstellung des Transportkoffers, die Bestückung mit den erforderlichen funktionalen Elementen sowie die korrekte Positionierung des Peritonealdialysegerätes umfassen.

Vorzugsweise entnimmt der Patient das Peritonealdialysegerät und das Organisationssystem aus dem Transportkoffer, baut das Organisationsystem auf, stellt das Peritonealdialysegerät auf den Transportkoffer und bestückt das Organisationsystem und/oder den Transportkoffer mit Lösungsbeuteln, Drainagebeutel, konnektiert das Schlauchsystem etc.

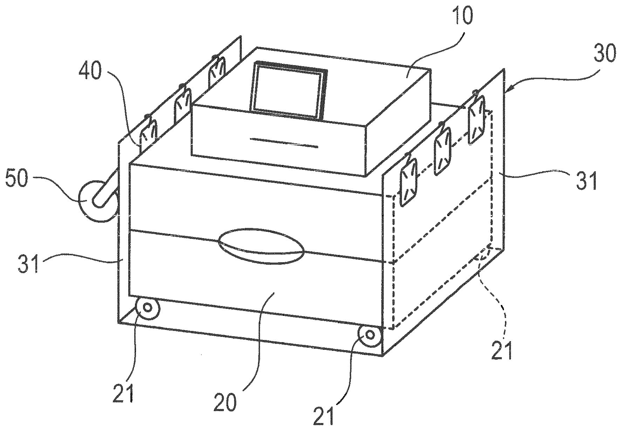

Weitere Einzelheiten und Vorteile werden anhand eines in der Zeichnung dargestellten Ausführungsbeispiels näher erläutert.

Die einzige Figur zeigt schematisch ein nicht unter die Erfindung fallendes Peritonealdialysesystem im aufgebauten Zustand.

Das Bezugszeichen 10 kennzeichnet ein APD-Peritonealdialysegerät, das auf dem Transportkoffer 20 aufliegt. Der Transportkoffer für das Peritonealdialysegerät dient in diesem Fall als Tisch für das Peritonealdialysegerät.

Mit dem Bezugszeichen 30 ist das faltbare Organisationssystem gekennzeichnet, dass im zusammengefalteten Zustand zusammen mit dem Peritonealdialysegerät in dem Transportkoffer 20 verstaut werden kann.

Wie dies aus der Figur ersichtlich ist, erstreckt sich das Organisationssystem 30 auf zwei gegenüberliegenden Seiten des Transportkoffers 20 und des Peritonealdialysegerätes 10 in Form von im Wesentlichen senkrecht stehenden Wandungen 31 bzw. Gestellteilen. Zur Stabilisierung von Wandungen kann das Organisationssystem weiterhin über eine Bodenplatte verfügen.

Die Wandungen/Gestellteile weisen eine Höhe auf, die die des liegenden Transportkoffers 20 übersteigt und die sich bis in den Bereich des Peritonealdialysegerätes 10 erstreckt. Der Transportkoffer 20 weist eine Oberseite, auf der das Peritonealdialysegerät steht und eine mit Rollen 21 versehene Unterseite auf. Somit lässt sich der Transportkoffer 20 mitsamt dem Peritonealdialysegerät 10 und dem Organisationssystem 30 bewegen, so dass eine für den Patienten optimale Position erreichbar ist.

An dem oberen Ende der Wandungen 31 befinden sich Haltemittel, wie z.B. Haken etc. an denen Lösungsbeutel 40 angehängt sind, die die frische, zu verabreichende Dialyselösung enthalten.

Diese stehen über ein nicht dargestelltes Schlauchsystem mit dem Peritonealdialysegerät in Verbindung. Das Peritonealdialysegerät fördert die frische Dialyselösung durch das Schlauchsystem zum nicht dargestellten Patientenkatheter, durch den diese in den Bauchraum des Patienten gelangt. Nach einer Verweildauer wird die Dialyselösung wieder abgepumpt und über den Patientenkatheter und ein nicht dargestelltes Schlauchsystem, das sich durch eine Öffnung in dem Koffer 20 hindurch erstreckt, in einen Drainagebeutel entleert. Dieser befindet sich in dem geschlossenen Koffer 20, auf dem das Peritonealdialysegerät steht.

Das Bezugszeichen 50 kennzeichnet einen an dem Organisationssystem befindlichen Organizer.

Die Vorteile der vorliegenden Erfindung bestehen vorzugsweise darin, dass das Organisationssystem leicht, portabel, platzsparend, kompakt ausgeführt ist und dass der Patient sich nicht umgewöhnen muss, wenn er unterwegs ist. Generell ist auch denkbar, dass dies bevorzugte Option für den Heimpatienten ist, beispielsweise bei beengten Platzverhältnissen im heimischen Umfeld, wie z.B. im Schlafzimmer.

Unterwegs und auch zu Hause können die gewohnten Abläufe beibehalten werden, was zu einer Verringerung möglicher Fehler bei der Anwendung bzw. Behandlung führt, die ggf. zu einer Peritonitis führen könnten.

Durch das Organisationssystem und/oder den Transportkoffer lassen sich vorzugsweise insbesondere Lösungsbeutel, das Drainagesystem und der Organizer sowie das PD bzw. APD-Gerät als solches an den gewünschten Stellen und auf der gewünschten Höhe positionieren, so dass eine reibungslose und störungsfreie Behandlung u.a. während der Nacht gewährleistet ist.

## Patentansprüche

1. Peritonealdialysesystem umfassend wenigstens ein auf Reisen transportierbares Peritonealdialysegerät sowie wenigstens ein Organisationssystem, das ausgebildet ist, das Peritonaeldialysegerät und ein oder mehrere funktionale Elemente, die mit dem Peritonealdialysegerät in Verbindung stehen oder mit diesem verbindbar sind, zu tragen, wobei das Organisationssystem zu Transport- oder Lagerzwecken zusammenfaltbar ist und zum Zwecke der Durchführung der Peritonealdialysebehandlung ausgehend von dem zusammengefalteten Zustand in seinen Funktionszustand aufbaubar ist, wobei das Organisationssystem eine höhenverstellbare Abstellfläche, auf der das Peritonealdialysgerät abstellbar ist, und ein oder mehrere Tragelemente zum Tragen des oder der funktionalen Elemente aufweist und wobei das Organisationssystem in seinem Funktionszustand das Peritonealdialysegerät teilweise oder vollständig umgibt und wobei das Peritonealdialysesystem einen Transportkoffer umfasst, in dem das Peritonealdialysegerät aufnehmbar ist und das Organisationssystem im zusammengefalteten Zustand Platz findet.

2. Peritonealdialysesystem nach Anspruch 1, **dadurch gekennzeichnet, dass** es sich bei dem oder den funktionalen Elementen um eines oder mehrere der folgenden Elemente handelt: Lösungsbeutel, Drainagebeutel, Schlauch, Ventil, Schlauchsystem, Organizer, Konnektor.

3. Peritonealdialysesystem nach einem der vorhergehenden Ansprüche, **dadurch gekennzeichnet, dass** das Organisationssystem Rollen aufweist, mittels derer das Organisationssystem verfahrbar ist und/oder dass das Organisationssystem höhenverstellbar ausgeführt ist.

4. Peritonealdialysesystem nach einem der vorhergehenden Ansprüche, **dadurch gekennzeichnet, dass** das Organisationssystem als von dem Transportkoffer getrennte Einheit ausgeführt ist.

5. Peritonealdialysesystem nach einem der vorhergehenden Ansprüche, **dadurch gekennzeichnet, dass** an dem Transportkoffer ein oder mehrere Stangen mit Halterungen und/oder sonstige Aufnahmen bzw. Halterungen für ein oder mehrere funktionale Elemente, insbesondere für Lösungsbeutel angeordnet sind.

6. Peritonealdialysesystem nach einem der vorhergehenden Ansprüche, **dadurch gekennzeichnet, dass** der Transportkoffer wenigstens eine Durchführung für zumindest einen Schlauch und wenigstens eine Aufnahme für wenigstens ein funktionales Element, insbesondere für einen Drainagebeutel und/oder für einen Lösungsbeutel aufweist.

7. Peritonealdialysesystem nach einem der vorhergehenden Ansprüche, **dadurch gekennzeichnet, dass** das Peritonealdialysesystem mit dem oder den funktionalen Elementen ausgestattet ist.

8. Verfahren zur Vorbereitung einer Peritonealdialysebehandlung, **dadurch gekennzeichnet, dass** zur Vorbereitung ein Peritonealdialysesystem gemäß einem der Ansprüche 1 bis 7 verwendet wird.

## Claims

1. Peritoneal dialysis system comprising at least one peritoneal dialysis machine that is transportable when travelling and at least one organization system that is configured to carry the peritoneal dialysis machine and/or one or more functional elements that are connected to the peritoneal dialysis machine or are connectable thereto, wherein the organization system can be folded for purposes of transport or storage and can be set up, starting from the folded state, into its functional state for the purpose of carrying out the peritoneal dialysis treatment, wherein the organization system has a height-adjustable placing surface, onto which the peritoneal dialysis machine can be placed, and one or more one or more carrier elements for carrying the functional elements and wherein the organization system partially or completely surrounds the peritoneal dialysis machine in its functional state and wherein the peritoneal dialysis system has a transport case in which the peritoneal dialysis machine can be received and in which is space for the organization system in its folded state.

2. Peritoneal dialysis system in accordance with claim 1, **characterized in that** the functional element or elements is/are one or more of the following elements: solution bags, drainage bags, hose, valve, hose system, organizer, connector.

3. Peritoneal dialysis system in accordance with one of the preceding claims, **characterized in that** the organization system has rollers by means of which the organization system is travelable; and/or **in that** the organization system is vertically adjustable.

4. Peritoneal dialysis system in accordance with one of the preceding claims, **characterized in that** the organization system is designed as a unit separate from the transport case.

5. Peritoneal dialysis system in accordance with one of the preceding claims, **characterized in that** one or more bars having holders and/or other receivers or holders for one or more functional elements, in particular for solution bags, are arranged at the transport case.

6. Peritoneal dialysis system in accordance with one of the preceding claims, **characterized in that** the transport case has at least one leadthrough for at least one hose and at least one receiver for at least one functional element, in particular for a drainage bag and/or for a solution bag.

7. Peritoneal dialysis system in accordance with one of the preceding claims, **characterized in that** the peritoneal dialysis system is equipped with the functional element or elements.

8. Method of preparing a peritoneal dialysis treatment, **characterized in that** a peritoneal dialysis system in accordance with one of the claims 1 to 7 is used for preparing a peritoneal dialysis system.

## Revendications

1. Système de dialyse péritonéale comprenant au moins un appareil de dialyse péritonéale transportable ainsi qu'au moins un système d'organisation qui est configuré pour porter l'appareil de dialyse péritonéale et un ou plusieurs éléments fonctionnels qui sont en liaison avec l'appareil de dialyse péritonéale ou qui peuvent être reliés à celui-ci, le système d'organisation pouvant être replié à des fins de transport ou de stockage et pouvant être monté à partir de l'état replié dans son état fonctionnel en vue de l'exécution du traitement de dialyse péritonéale, le système d'organisation présentant une surface de pose réglable en hauteur, sur laquelle l'appareil de dialyse péritonéale peut être posé, et un ou plusieurs éléments porteurs pour porter le ou les éléments fonctionnels, et le système d'organisation, dans son état fonctionnel, entourant partiellement ou complètement l'appareil de dialyse péritonéale, et le système de dialyse péritonéale comprenant une valise de transport dans laquelle l'appareil de dialyse péritonéale peut être logé et le système d'organisation trouve place à l'état replié.

2. Système de dialyse péritonéale selon la revendication 1, **caractérisé en ce que** le ou les éléments fonctionnels consistent en un ou plusieurs des éléments suivants : poche de solution, poche de drainage, tubulure, valve, système de tubulure, organisateur, connecteur.

3. Système de dialyse péritonéale selon l'une quelconque des revendications précédentes, **caractérisé en ce que** le système d'organisation présente des roulettes au moyen desquelles le système d'organisation peut être déplacé et/ou **en ce que** le système d'organisation est conçu de manière à être réglable en hauteur.

4. Système de dialyse péritonéale selon l'une quelconque des revendications précédentes, **caractérisé en ce que** le système d'organisation est conçu sous forme d'unité séparée de la valise de transport.

5. Système de dialyse péritonéale selon l'une quelconque des revendications précédentes, **caractérisé en ce qu'**une ou plusieurs barres avec des supports et/ou d'autres logements ou supports pour un ou plusieurs éléments fonctionnels, notamment pour des poches de solution, sont agencés sur la valise de transport.

6. Système de dialyse péritonéale selon l'une quelconque des revendications précédentes, **caractérisé en ce que** la valise de transport présente au moins un passage pour au moins une tubulure et au moins un logement pour au moins un élément fonctionnel, notamment pour une poche de drainage et/ou pour une poche de solution.

7. Système de dialyse péritonéale selon l'une quelconque des revendications précédentes, **caractérisé en ce que** le système de dialyse péritonéale est équipé du ou des éléments fonctionnels.

8. Procédé de préparation d'un traitement de dialyse péritonéale, **caractérisé en ce que,** pour la préparation, un système de dialyse péritonéale selon l'une quelconque des revendications 1 à 7 est utilisé.
